# EUROPEAN PATENT APPLICATION

(11) **EP 2 859 837 A1**
(43) Date of publication of application: **15.04.2015**
(21) Application number: 13804945.7
(22) Date of filing: 27.05.2013
(51) Int. Cl.: A61B 1/06, A61B 1/04, G02B 21/06, G02B 23/24, H04N 5/225, H04N 5/238, H04N 7/18

(54) **IMAGING DEVICE, MICROSCOPE DEVICE, AND ENDOSCOPE DEVICE**

(30) Priority: 12.06.2012 JP 2012133175
(71) Applicant: Olympus Corporation, Shibuya-ku Tokyo 151-0072 (JP)
(72) Inventor: YAMAMOTO, Eiji, Hachioji-shi Tokyo 192-8512 (JP); SHIMIZU, Hatsuo, Hachioji-shi Tokyo 192-8512 (JP); ITO, Takeshi, Hachioji-shi Tokyo 192-8512 (JP)
(74) Representative: Winter, Brandl, Fürniss, Hübner, Röss, Kaiser, Polte - Partnerschaft mbB
(86) International application number: PCT/JP2013/064576
(87) International publication number: WO 2013/187215

(57) **Abstract**

An imaging apparatus (100) includes an imaging unit (102), an illuminating unit (104), and an image-characteristic setting unit (106). The imaging unit (102) images an object to acquire an image of the object. The illuminating unit (104) includes a light source configured to apply illumination light beams different in optical characteristics to the object. The image-characteristic setting unit (106) sets the image characteristics of the image data the imaging unit (102) should acquire, then refers to light source characteristic information including at least one of the light-intensity control characteristic data, light-distribution pattern characteristic data, spectrum distribution characteristic data and polarization characteristic data, and sets to the illuminating unit (104), the intensity, distribution pattern, spectrum distribution or polarization characteristic of at least one illumination light beam. The imaging unit (102) acquires effectively image data having the image characteristics set.

## Description

### Technical Field

This invention relates to an imaging apparatus, a microscope apparatus and an endoscope apparatus, each designed to apply light beams of different spectrum distributions to an object, thereby providing images different in characteristics.

### Background Art

Imaging apparatuses are known which apply light beams of different spectrum distributions to an object, thereby providing images different in characteristics. Jpn. Pat. Appln. KOKAI Publication No. 2005-13611, for example, discloses the technique of switching the illumination light for the object, from one to another, thereby providing images of various characteristics for use in various imagings such as ordinary imaging, fluorescence imaging, narrow-band imaging and infrared imaging. Jpn. Pat. Appln. KOKAI Publication No. 2005-13611 further discloses that a data holding means is used, which holds circuit data that a programmable logic-element circuit of small scale may use to process the images of different characteristics, and that the circuit data is selected from the data holding means in accordance with the characteristic of the image.

### Summary of the Invention

In the imaging apparatus of Jpn. Pat. Appln. KOKAI Publication No. 2005-13611, images different in characteristics are acquired by switching the spectrum of the light illuminating the object, from one to another. In the apparatus, a process such as color conversion for acquring an image of a desirable characteristic is performed in a logic circuit provided at the output of the imaging element. This process need not be performed or can be simplified if the object is illuminated with light of optimal illumination characteristics (e.g., intensity, light distribution pattern and spectrum distribution) and if the imaging element thereby generates an image of the desirable characteristic. In this case, the image processing circuit incorporated in the imaging apparatus can be made smaller, ultimately miniaturizing the imaging apparatus, lowering the manufacturing cost and reducing the power consumption.

This invention has been made in consideration of the above. An object of the invention is to provide an imaging apparatus that illuminates an object with light having an optimal characteristic to obtain an image of a desirable characteristic without requiring complex image processing, and to provide a microscope apparatus and an endoscope apparatus, each comprising the imaging apparatus.

According to an aspect of the invention, an imaging apparatus comprises: an imaging unit configured to image an object to acquire image data about the object; an illuminating unit including a light source configured to apply, to the object, a plurality of illumination light beams different in optical characteristics; and an image-characteristic setting unit configured to set image characteristics of the image data the imaging unit should acquire, to refer to light source characteristic information including at least one of light-intensity control characteristic data, light-distribution pattern characteristic data, spectrum distribution characteristic data and light-polarization characteristic data, and to set to the illuminating unit, intensity, distribution pattern, spectrum distribution or polarization characteristic of at least one illumination light beam, thereby enabling the imaging unit to acquire effectively image data having the image characteristics set.

### Brief Description of Drawings

FIG. 1 is a block diagram schematically showing the configuration common to imaging apparatuses according to embodiments of this invention;
FIG. 2 is a diagram showing, in detail, the configuration of an imaging apparatus according to a first embodiment of the invention;
FIG. 3A is a first diagram explaining how the imaging apparatus according to the first embodiment of the invention operates;
FIG. 3B is a second diagram explaining how the imaging apparatus according to the first embodiment of the invention operates;
FIG. 3C is the third diagram explaining how the imaging apparatus according to the first embodiment of the invention operates;
FIG. 4 is a diagram showing, in detail, the configuration of an imaging apparatus according to a second embodiment of the invention;
FIG. 5 is a diagram showing the light-distribution patterns and spectrum distributions of the light sources constituting a light source module;
FIG. 6 is a diagram explaining how an imaging apparatus according to a second embodiment of the invention operates;
FIG. 7 is a diagram showing, in detail, the configuration of an imaging apparatus according to a third embodiment of the invention;
FIG. 8A is a first diagram explaining how the imaging apparatus according to the third embodiment of the invention operates;
FIG. 8B is the second diagram explaining how the imaging apparatus according to the first embodiment of the invention operates;
FIG. 9 is a block diagram showing the configuration of an imaging apparatuses according to a fourth embodiment of this invention;
FIG. 10A is a first diagram explaining how the imaging apparatus according to the fourth embodiment of the invention operates;
FIG. 10B is the second diagram explaining how the imaging apparatus according to the fourth embodiment of the invention operates;
FIG. 11 is a block diagram showing the configuration of an imaging apparatus according to a fifth embodiment of this invention;
FIG. 12 is a diagram showing an exemplary configuration of a light source module; and
FIG. 13 is a diagram showing an exemplary configuration of a light source module using a variable focal-length optical system.

### Description of Embodiments

Embodiments of this invention will be described with reference to the accompanying drawing.

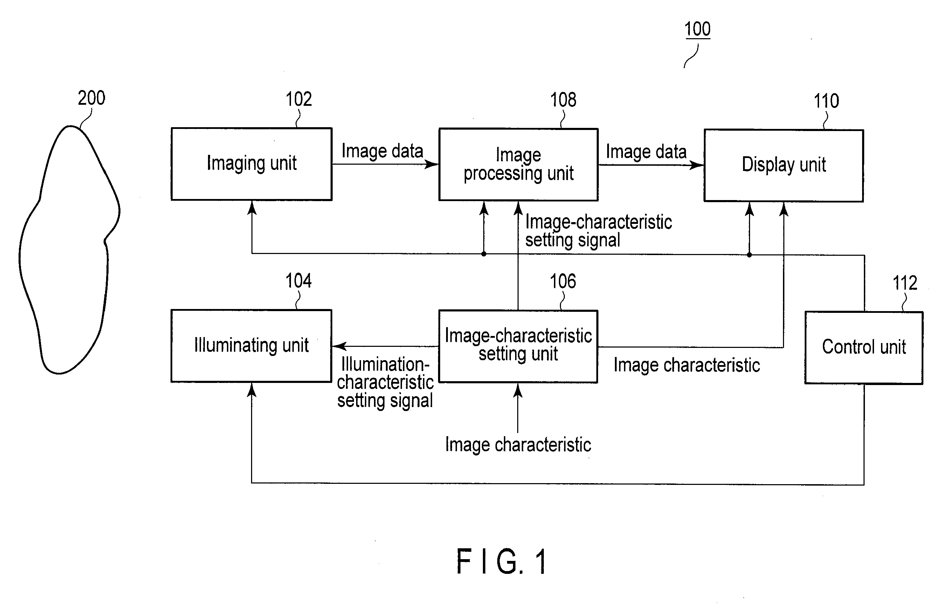

FIG. 1 is a block diagram schematically showing the configuration common to imaging apparatuses 100 according to embodiments of the invention. As FIG. 1 shows, any imaging apparatus according to this invention has an imaging unit 102, an illuminating unit 104, an image-characteristic setting unit 106, an image processing unit 108, a display unit 110, and a control unit 112.

The imaging unit 102 images an object 200 to generate digital image data about the object 200.

The illuminating unit 104 is the light source for illuminating the object 200. The illuminating unit 104 has a light source that can emit a plurality of illumination light beams different in light source characteristic. The "light source characteristic" includes at least one selected from the group consisting of a light distribution pattern, spectrum distribution, and state of polarized light. The light distribution pattern represents the angle and intensity at which illumination light is applied to the object. The spectrum distribution represents that the illumination light includes the light of which waveband. The illuminating unit 104 is programmed to set the spectrum of the illumination light emitted from the light source, the light distribution pattern, polarization characteristic, light intensity, number of times light has been emitted and light emission timing, in accordance with an illumination-characteristic setting signal supplied from the image-characteristic setting unit 106.

The image-characteristic setting unit 106 programs the illuminating unit 104 and image processing unit 108, so that the imaging unit 102 may effectively acquire image data having desirable image characteristics. The image characteristics are information representing the characteristics of the image data. This information includes, for example, the information representing the band (color tone or spectrum band) that should be emphasized or extracted in the image data, the information representing the region to illuminate with illumination light, and the information representing the dynamic range of the image data. The word "effectively" means that an image of a desirable characteristic can be acquired in a simple process performed at the output of the imaging unit 102.

The image processing unit 108 processes the image data received from the imaging unit 102, converting the same to data that can be played back. This image processing is, for example, gamma correction. The image processing unit 108 further synthesizes, if necessary, the image data generated in the imaging unit 102.

The display unit 110 displays the image represented by the image data processed in the image processing unit 108. The display unit 110 displays also various information items such as the image characteristic set in the image-characteristic setting unit 106.

The control unit 112 inputs a sync signal to the imaging unit 102, illuminating unit 104, image processing unit 108 and display unit 110, controlling these units 102, 104, 108 and 110 in synchronism.

The embodiments of this invention will be described in detail.

### [First Embodiment]

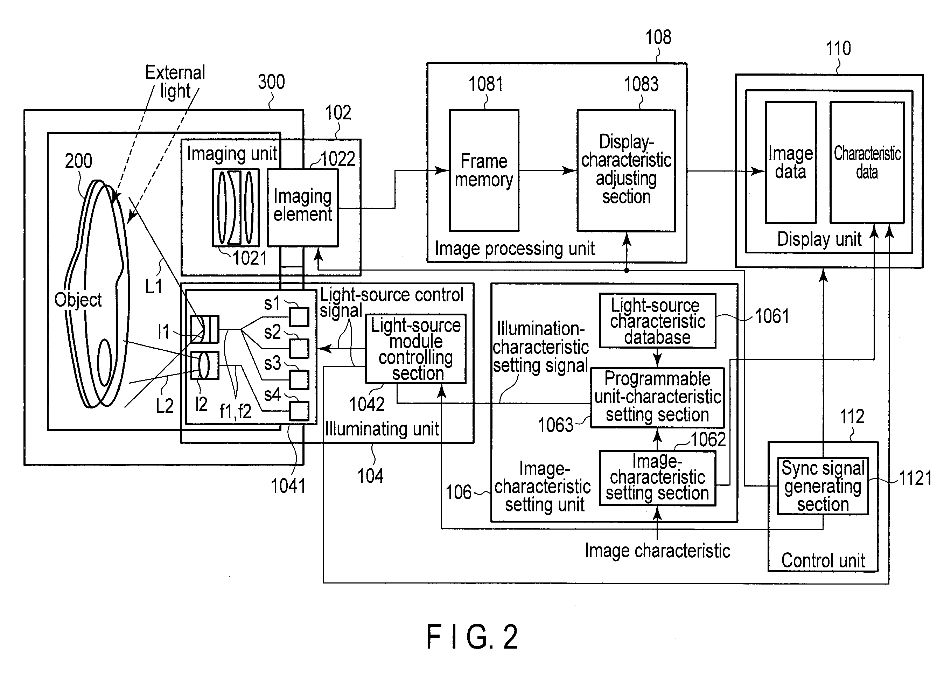

FIG. 2 is a diagram showing, in detail, the configuration of an imaging apparatus according to the first embodiment of the invention. In this embodiment, the object 200 is preferably imaged and illuminated by the imaging unit 102 and illuminating unit 104, respectively, in such an environment where the external light applied to the object 200 is negligibly weak with respect to the illumination light applied to the object 200 from the illuminating unit 104. It is therefore desired that the imaging unit 102 and illuminating unit 104 image and illuminate the object 200 in, for example, an external-light shielding member 300.

The external-light shielding member 300 is a component that provides an environment in which the external light applied to the object 200 is, in effect, negligibly weak with respect to the illumination light applied to the object 200 from an illuminating means. As shown in FIG. 2, the external-light shielding member 300 is shaped like a box, enclosing the imaging unit 102, illuminating unit 104 and object 200. The external-light shielding member 300 may be made, as needed, of a material that reflects or absorbs external light.

The external-light shielding member 300 may not be used. In this case, a process is performed for cancelling the external light. Two alternative processes may be performed to cancel the external light in the case where the external light or the light emitted by the light source module 1041 is applied to the object 200 at a preset spectrum, in a preset cycle or at a preset time. In one method, the spectral component of the external light or the illumination-cycle component or illumination-timing component of the external light is eliminated, thereby cancelling the external-light component in the image data acquired in the imaging unit 102. In the other method, the spectral component of the illumination light or the illumination-cycle component or illumination-timing component of the illumination light is extracted, electrically or by using software, thereby extracting the illumination-light component.

The imaging unit 102 has an imaging optical system 1021 and an imaging element 1022.

The imaging optical system 1021 has one or more lenses, and focuses the light reflected, scattered or diffracted by the object 200, at the imaging element 1022. The imaging element 1022 has a light-receiving surface, at which photoelectric converting elements are arranged, and converts the light coming from the object 200 through the imaging optical system 1021 and then focused, to an analog electric signal (image signal). The imaging element 1022 has an A/D converting circuit (not shown). The A/D converting circuit converts the analog electric signal (image signal) to image data that is a digital signal.

The illuminating unit 104 has a light source module 1041 and a light-source module controlling section 1042.

The light source module 1041 has one or more light sources for emitting light beams different in light distribution pattern and spectrum distribution. The light source module 1041 shown in FIG. 2 has four light sources, s1 to s4. The light sources s1 to s4 have different spectrum distributions. The light sources s1 to s3 are connected to a light guiding path f1 composed of, for example, an optical fiber. The light source s4 is connected to a light guiding path f2. The light guiding path f1 is connected to an illumination optical system 11. The light guiding path f2 is connected to an illumination optical system 12. In the imaging apparatus shown in FIG. 2, the illumination optical system 11 has characteristics for distributing illumination light in a wide-angle range, and the illumination optical system 12 has characteristics for distributing illumination light in a narrow-angle range. Since illumination optical systems having different characteristics are provided in the light source module 1041, the light sources s1 to s3 can have one light distribution pattern, and the light source s4 can have another light distribution pattern.

In the embodiment of FIG. 2, the light source module 1041 incorporates four light sources. The number of light sources is not limited. Further, the light sources connected to the light guiding path f1 may be switched among them. For example, any one of the light sources s1 to s3 may be connected to the light guiding path f1, or any two of the light sources s1 to s3 may be connected to the light guiding path f1. In this embodiment, optical fibers are used as light guiding paths. The light guiding paths are not limited to optical fibers. Any other members may be used instead, provided they transmit light. Optical waveguides, for example, may be used instead. Moreover, the number of illumination optical systems is not limited to two. Only one illumination optical system may be used if it is, for example, a variable-power optical system.

The light-source module controlling section 1042 combines the light beams emitted from the light sources s1 to s4 in a light guiding path or modulates these light beams by using an optical modulation element (not shown). Thus, the light-source module controlling section 1042 controls the illumination light emitted from the illuminating unit 104, in terms of at least one of the light intensity, distribution pattern, spectrum distribution and polarization characteristic.

The image-characteristic setting unit 106 has a light-source characteristic database 1061, an image-characteristic setting section 1062, and a programmable unit-characteristic setting section 1063.

The light-source characteristic database 1061 holds, in the form of a database, the light-source characteristic information about the light source module 1041, such as the spectrum-distribution characteristic information about the light sources s1 to s4 constituting the light source module 1041 and the distribution-pattern characteristic information based on the characteristics of the illumination optical systems 11 and 12.

The image-characteristic setting section 1062 sets the image characteristics the imaging unit 102 acquires as the illuminating unit 104 applies light to the object 200. More precisely, the image characteristics are set at the image-characteristic setting section 1062 by, for example, the user.

The programmable unit-characteristic setting section 1063 refers to the light-source characteristic database 1061 in accordance with the image characteristics set at the image-characteristic setting section 1062, generating an illumination-characteristic setting signal for generating image data of desirable characteristic in the imaging unit 102. The illumination-characteristic setting signal is input to the illuminating unit 104. How the programmable unit-characteristic setting section 1063 operates will be explained later in detail.

The image processing unit 108 has a frame memory 1081 and a display-characteristic adjusting section 1083.

The frame memory 1081 temporarily stores the image data acquired in the imaging unit. The display-characteristic adjusting section 1083 performs a process, such as gamma correction, on the image data read from the frame memory 1081, in accordance with the characteristics of the display unit 110.

The display unit 110 comprises a display such as a liquid crystal display, and displays the image represented by the image data processed in the display-characteristic adjusting section 1083. The display unit 110 may display an information window for displaying the information representing the image characteristics set at the image-characteristic setting unit 106, the information representing the setting of the light source module 104 and the various information items held in the light-source characteristic database 1061.

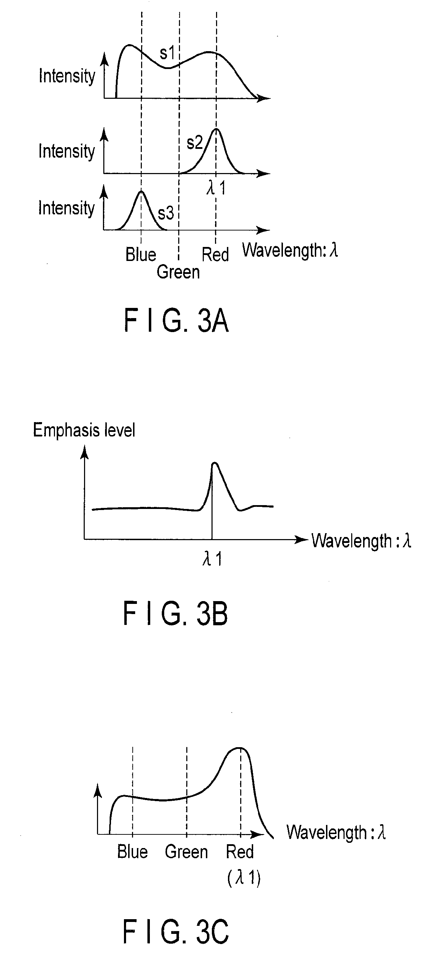

How the imaging apparatus shown in FIG. 2 operates will be explained. A case will be explained wherein the imaging unit 102 acquires image data which has an image characteristic in which a particular spectral band is emphasized. In this case, the light sources s1 to s3 are used, and the light source s4 is not used. FIG. 3A shows the spectrum distributions of the light beams emitted from the light sources s1 to s3. As seen from FIG. 3A, the light source s1 emits light having a continuous white spectrum over the visible band, the light source s2 emits light having the spectral peak in the red wavelength band, and the light source s3 emits light having the spectral peak in the blue wavelength band. The light source s4 emits light having the spectral peak in, for example, a specific narrow wavelength band (hereinafter, this light will be referred to as "special light"). The light source s4 will be later described in detail in connection with the second embodiment.

The information about the spectral band to emphasize is set in the image-characteristic setting section 1062. Assume that as shown in FIG. 3B, a band near wavelength λ1 (e.g., red wavelength band), for example, is set as the spectral band to emphasize. Then, the programmable unit-characteristic setting section 1063 refers to the light-source characteristic database 1061 and selects a light source and sets the intensity ratio of the light to emit from the light source, and generates an image-characteristic setting signal in accordance with this setting.

The information showing that the light sources s1 to s3 have such spectrum distributions as shown in FIG. 3A may be acquired from the light-source characteristic database 1061. If this is the case, the programmable unit-characteristic setting section 1063 inputs an illumination-characteristic setting signal to the light source module 1041, instructing that the light emitted by the light source s2, i.e., source of red-wavelength light, should be set to a higher intensity than light beams emitted from the light sources s1 and s3 in order to emphasize red in the image data.

On receiving the illumination-characteristic setting signal, the light-source module controlling unit 1042 generates a light-source control signal. The light-source control signal is output to the light sources s1 and s2, which emits light beams at intensities preset. The light sources s3 and s4 do not emit light.

In synchronism with the sync signal coming from the control unit 112, the light source module 1041 makes the light sources s1 and s2 emit light at the intensity designated by the light-source control signal. The light beams emitted from the light sources s1 and s2 are combined in the light guiding path f1, providing illumination light L1. The illumination light L1 is applied to the object 200. Since the output light of the light source s2 is intensified, the illumination light L1 applied to the object 200 has such a spectrum distribution with the red wavelength band so emphasized as shown in FIG. 3C.

In this instance, the light sources s1 and s2 emit light at the same time. Instead, the light sources s1 and s2 may be sequentially driven at short interval to emit two light beams one after the other. In this case, too, light having an emphasized spectrum distribution can be regarded as applied to the object 200.

In response to the sync signal supplied from the control unit 112, the imaging unit 102 images the object 200 at the same time the light source module 104 emits illumination light, generating image data. Since the object 200 is illuminated with the illumination light L1 at the time of imaging, the image data acquired in the imaging unit represents a low color temperature (namely, red is emphasized).

The image data acquired in the imaging unit 102 is temporarily stored in the frame memory 1081 and then read by the display-characteristic adjusting section 1083. The display-characteristic adjusting section 1083 performs a process, such as gamma correction, on the image data read from the frame memory 1081. The image data so processed is input to the display unit 110. The display unit 110 displays the image represented by the image data input to it. At this point, the display unit 110 displays, if necessary, the image characteristic, too. Thus ends the sequence of processes, from the imaging of the object 200 to the displaying of the image of the object 200.

In the sequence of processes, the image processing unit 108 need not perform an image processing to emphasize the image in the specific narrow wavelength band. In the instance of FIGS. 3A to 3C, the display unit 110 displays an image in which to emphasize the red component. If the light emitted from the light source s3 is intensified, the display unit 110 can display an image emphasized for the blue component (or having a blue component with a high color temperature). Further, the ratio of the intensity of the light source s1 to that of the light source s3 may be changed, thereby adjusting the color balance in the image data.

As described above, the illuminating unit 104 can be programmed (in terms of the combination of light sources and the light intensity, i.e., light amount) by referring to the light-source characteristic information stored in the light-source characteristic database, thereby illuminating the object 200 in such conditions that the imaging unit 102 can generate image data representing an image of a desirable color. The number of image processing steps the image processing unit 108 performs can therefore be reduced. As a result, the image processing unit 108 can be simplified in configuration.

In the first embodiment, the illuminating unit 104 may be configured to be replaced by another illuminating unit. Then, the imaging unit 102 can acquire mage data having more image characteristics. In this case, however, the programmable unit-characteristic setting section 1063 needs to acquire the light-source characteristic information about the replacement illuminating unit.

### [Second Embodiment]

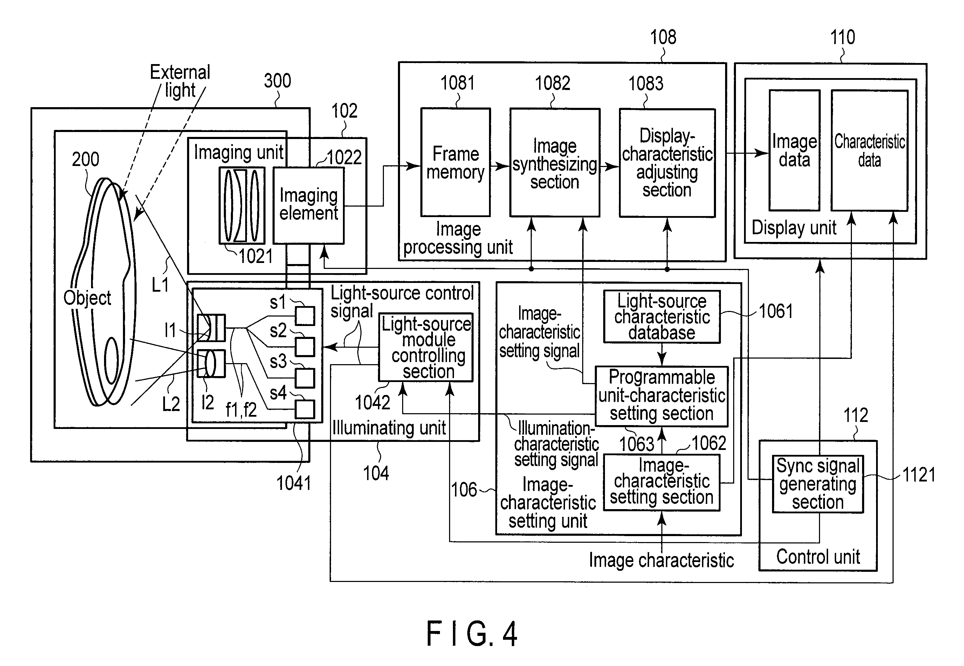

The second embodiment of this invention will be described. FIG. 4 is a diagram showing, in detail, the configuration of an imaging apparatus according to the second embodiment of the invention. The configuration features different from those shown in FIG. 2 will be described, and the features common to the first embodiment will not be described.

In the second embodiment, the programmable unit-characteristic setting section 1063 generates an illumination-characteristic setting signal and an image-characteristic setting signal in accordance with the image characteristics set at the image-characteristic setting section 1062. The illumination-characteristic setting signal is input to the light-source module controlling section 1042. The image-characteristic setting signal is input to an image synthesizing section 1082, which is incorporated in the image processing unit 108.

That is, the image processing unit 108 has the image synthesizing section 1082, in addition to the frame memory 1081 and display-characteristic adjusting section 1083. The image synthesizing section 1082 synthesizes the image data stored in the frame memory 1081 in accordance with the image-characteristic setting signal input from the programmable unit-characteristic setting section 1063. As will be described later in detail, this embodiment performs a process of synthesizing image data for a plurality of frames different in illumination state, thereby generating more various image data in image characteristics than those in the first embodiment. The frame memory 1081 therefore has a storage capacity for storing image data sufficient for several frames at the same time.

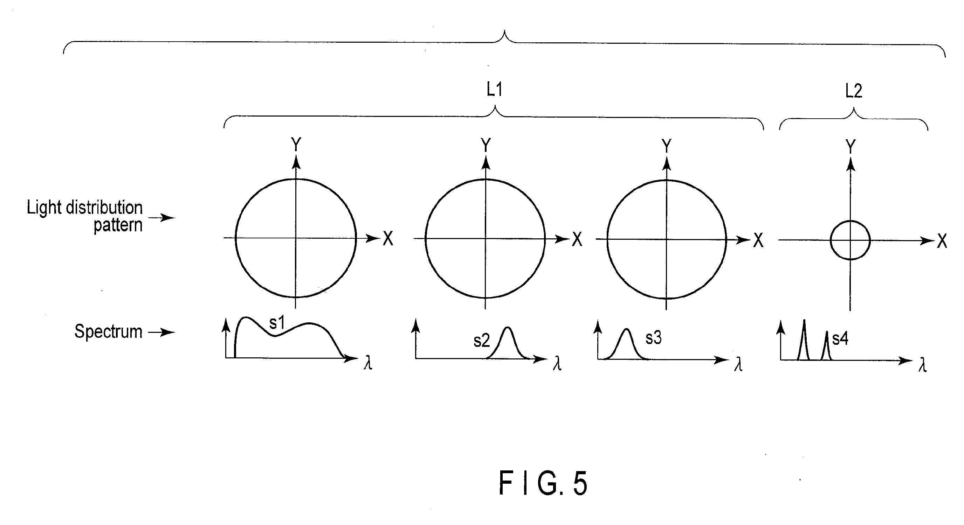

How the imaging apparatus shown in FIG. 4 operates will be explained. First, it will be described how to switch the illumination pattern to acquire image data items and how to synthesize the image data items to generate image data having desirable image characteristics. FIG. 5 shows the light-distribution patterns and spectrum distributions of the light sources s1 to s4. In FIG. 5, the light-distribution patterns are illustrated with respect to directions X and Y intersecting at right angles in a plane perpendicular to the axis of the illumination light beam emitted from the illumination optical system 11 or 12.

As described above, the light source s1 emits light having a continuous white spectrum over the visible band, the light source s2 emits light having the spectral peak at the red wavelength band, and the light source s3 emits light having the spectral peak at the blue wavelength band. The light source s4 emits special light. The special light is utilized to achieve fluorescence analysis in biochemical research and medical diagnosis, or to provide narrow spectrum-band images for medical diagnosis.

As specified above, the illumination optical system 11 has characteristics for distributing illumination light in a wide-angle range, and the illumination optical system 12 has characteristics for distributing illumination light in a narrow-angle range.

In this embodiment, the object is imaged for several frames and finally acquiring image data of the following four characteristic types:
Image data of a large dynamic range for brightness
Image data of low color temperature (red emphasized)
Image data of high color temperature (blue emphasized)
Image data acquired by applying special narrow-band light to a narrow area

If the image-characteristic setting section 1062 is set to acquire image data of these four characteristic types, the programmable unit-characteristic setting section 1063 refers to the light-source characteristic database. As described above, the programmable unit-characteristic setting section 1063 selects a light source in the illuminating unit 104, sets the intensity ratio of the light to emit from the light source, and generates an image-characteristic setting signal in accordance with this setting.

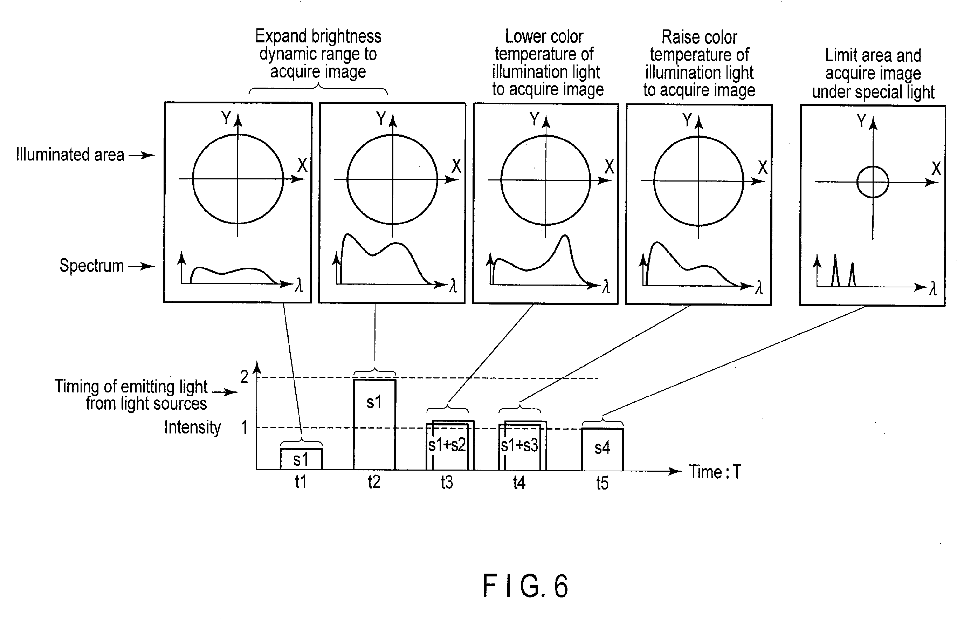

The light sources s1 to s4 may have, for example, such light distribution patterns and spectrum distributions as shown in FIG. 5. In this case, the programmable unit-characteristic setting section 1063 inputs, at time t1 (see FIG. 6), an illumination-characteristic setting signal to the light-source module controlling section 1042, instructing that the light emitted by the light source s1 be set to low intensity (e.g., half). At time t2 (FIG. 6), the programmable unit-characteristic setting section 1063 inputs an illumination-characteristic setting signal to the light-source module controlling section 1042, instructing that the light emitted by the light source s1 be set to high intensity (e.g., two times). At time t3 (FIG. 6), the programmable unit-characteristic setting section 1063 inputs an illumination-characteristic setting signal to the light-source module controlling section 1042, instructing that the light sources s1 and s2 should emit light at the same time, at normal intensity (even). At time t4 (FIG. 6), the programmable unit-characteristic setting section 1063 inputs an illumination-characteristic setting signal to the light-source module controlling section 1042, instructing that the light sources s1 and s3 should emit light at the same time, at normal intensity (even). At time t5 (FIG. 6), the programmable unit-characteristic setting section 1063 inputs an illumination-characteristic setting signal to the light-source module controlling section 1042, instructing that the light source s4 should emit light at normal intensity (even).

The programmable unit-characteristic setting section 1063 inputs an image-characteristic setting signal to the image synthesizing section 1082, instructing that the image data 1 and image data 2, acquired at time t1 and time t2, respectively, should be synthesized.

In accordance with the sync signal supplied from the control unit 112, the imaging unit 102 performs imaging at five frames synchronizing illumination pattern switching timings t1, t2, t3, t4, and t5. As a result, five image data 1, 2, 3, 4, and 5 are acquired.

The 5-frame image data acquired in the imaging unit 102 is temporarily stored in the frame memory 1081 and then input to the image synthesizing section 1082. The image synthesizing section 1082 has received an image-characteristic setting signal. As instructed by this signal, the image synthesizing section 1082 synthesizes the image data 1 and the image data 2, both input from the frame memory 1081, generating synthesized image data. The synthesized image data is output to the display-characteristic adjusting section 1083. The image synthesizing section 1082 outputs the image data 3, image data 4 and image data 5, all input from the frame memory 1081, to the display-characteristic adjusting section 1083, without processing them at all.

In the process that the image synthesizing section 1082 performs to expand the dynamic range, those parts of the image data 1 image data 2 which have prescribed brightness are synthesized. The dynamic range for brightness can thereby be expanded.

The display-characteristic adjusting section 1083 performs a process, such as gamma correction, on the image data read from the frame memory 1081. The image data so processed is input to the display unit 110. The display unit 110 displays the image represented by the image data input to it. At this point, the display unit 110 displays, if necessary, the image characteristic, too. Thus ends the sequence of processes, from the imaging of the object 200 to the displaying of the image of the object 200. The four images may be displayed at the same time, or one at a time.

As the sequence of processes described above proceeds, the display unit 110 displays images of various types, such as an image acquired by synthesizing image data 1 and image data 2, an image having an expanded dynamic range, an image corresponding to image data 3 and red-emphasized (having low color temperature), an image corresponding to image data 4 and blue-emphasized (having high color temperature), and an image corresponding to image data 5 defined by special light reflected, scattered or diffracted in a narrow area and containing fluorescent light.

As described above, the image synthesizing section 1082 adds the image data items acquired to generate synthesized image data. Nonetheless, the image synthesizing unit 1082 can perform various image-synthesizing operations. For example, the image data 5 (special light image) may be multiplied by a specific ratio and then subtracted from the image data 2 (white image), thus synthesizing the image data. In this case, image data excluding the special spectrum band only can be extracted.

As has been described, this embodiment, which has the image synthesizing section 1082, can generate more various image data in characteristics than those in the first embodiment. Moreover, this embodiment can acquire, through an imaging sequence, image data having several image characteristics by using an illumination-characteristic setting signal and an image-characteristic setting signal, in accordance with how the desirable image characteristic changes with time.

Image data having desirable image characteristics can be acquired by switching the illumination pattern in this embodiment, depending on the image characteristic, by using not only the method described above, but also some other methods. Some typical methods are as follows:
(1) To acquire image data having an expanded dynamic range as a desirable image characteristic:
   In order to acquire image data having an expanded dynamic range, a brightness dynamic range is set in the image-characteristic setting section 1062 of the image-characteristic setting unit 106. To expand the dynamic range, illumination light is applied from the light source to the object 200 several times, changing the intensity (amount) of light each time, and the imaging unit 102 images the object 200 so illuminated with the illumination light. The image data items acquired in the imaging unit 102 and differing in brightness are synthesized in the image synthesizing section 1082 of the image processing unit 108. A synthesized image having the desirable dynamic range is thereby acquired.
(2) To acquire image data having, as a desirable image characteristic, a particular wavelength band emphasized or suppressed:
   In order to acquire image data having a particular wavelength band emphasized or suppressed, the wavelength band to emphasize or suppress is set in the image-characteristic setting section 1062 of the image-characteristic setting unit 106. Several methods may be used to acquire image data in which the wavelength band is emphasized or extracted (or the color temperature or color tone is adjusted).

In a first method, which is identical to the method described above, the light beams emitted from several light sources different in spectrum are synthesized and light intensified in a particular wavelength band is applied to the object 200.

In a second method, the illumination light emitted from a light source (e.g., light source s1) having a broad spectrum distribution and the illumination light emitted from a light source (e.g., light source s2 or s3) having a spectral peak in a particular wavelength band are alternately applied to the object 200, and the imaging unit 102 images the object 200 so illuminated. The image data items acquired in the imaging unit 102 are added or synthesized, providing an image emphasized in a particular wavelength band. Conversely, the image data items may be subtracted one from another, thereby acquiring an image suppressed in a particular wavelength band.

In both the first method and the second method, a plurality of light sources are used. Instead, the illumination light emitted from one light source may be modulated by, for example, a light modulating element.
(3) To acquire image data having, as a desirable image characteristic, brightness emphasized at a particular area of the object:

In order to acquire image data having brightness emphasized at a particular area of the object, a target area to emphasize in brightness is set in the image-characteristic setting section 1062 of the image-characteristic setting unit 106. Further, a plurality of light sources of different distribution patterns are used or the illumination area of each illumination optical system is made variable, and at least one light source or one illumination optical system is selected to apply illumination light to the target area. Still further, the light beams emitted from the light sources different in light distribution pattern may be combined to acquire image data representing an image having a particular area brightened. The technique of emphasizing the brightness of a particular area of the object is effective, particularly in the case where light of a specific wavelength should be concentrated and applied to the particular area as in, for example, analyzing fluorescence, if the object is far from the illuminating unit 104 or if the object is too close to the illuminating unit 104 and excessively illuminated (possibly resulting in blown out highlights).

### [Third Embodiment]

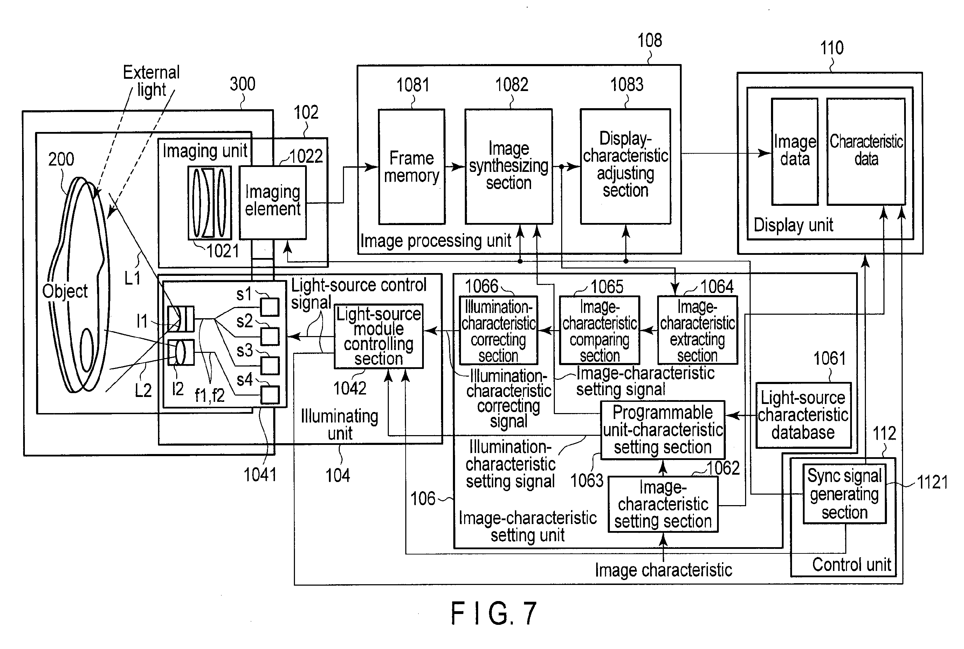

FIG. 7 is a diagram showing, in detail, the configuration of an imaging apparatus according to a third embodiment of the invention. The configuration features different from those shown in FIG. 4 will be described, and features common to the first embodiment will not be described.

As shown in FIG. 7, the image-characteristic setting unit 106 has an image-characteristic extracting section 1064, an image-characteristic comparing section 1065, and an illumination-characteristic correcting section 1066, in addition to the light-source characteristic database 1061, image-characteristic setting section 1062, and programmable unit-characteristic setting section 1063.

The image-characteristic extracting section 1064 extracts the image characteristic set in the image-characteristic setting section 1062, from the image data processed in the image synthesizing section 1082. The image characteristic may be set a particular spectrum band to emphasize. In this case, the image-characteristic extracting section 1064 extracts, as an image characteristic, the spectrum distribution or color temperature. In the third embodiment, the image synthesizing section need not be provided in the imaging apparatus 100. If the image synthesizing unit 1082 is not provided, the image-characteristic extracting section 1064 extracts the image characteristic from the image data stored in the frame memory 1081.

The image-characteristic comparing section 1065 compares the image characteristic extracted in the image-characteristic extracting section 1064 with the image characteristic set in the image-characteristic setting section 1062. More specifically, the image-characteristic comparing section 1065 calculates the difference between the image characteristic extracted in the image-characteristic extracting section 1064 and the image characteristic set in the image-characteristic setting section 1062.

The illumination-characteristic correcting section 1066 generates an illumination-characteristic correcting signal from the difference calculated by the image-characteristic comparing section 1065. The illumination-characteristic correcting signal is input to the light-source module controlling section 1042 in order to eliminate the difference between the image characteristic extracted in the image-characteristic extracting section 1064 and the image characteristic set in the image-characteristic setting section 1062.

How the imaging apparatus of FIG. 7 operates will be explained. In accordance with the image characteristic set in the image-characteristic setting section 1062, the programmable unit-characteristic setting section 1063 sets the light-source module controlling section 1042 and image synthesizing section 1082.

Set by the programmable unit-characteristic setting section 1063, the light-source module controlling section 1042 controls the light source module 1041, thereby illuminating the object 200 with illumination light.

At the same time the object 200 is illuminated, the imaging unit 102 images the object 200 to generate image data. The image data is temporarily stored in the frame memory 1081 and then read to the image synthesizing section 1082. The image synthesizing section 1082 synthesizes image data when it is set by the programmable unit-characteristic setting section 1063.

The image-characteristic extracting section 1064 extracts the image characteristic set by the image-characteristic setting section 1062, from the image data output from the image synthesizing section 1082. The image-characteristic comparing section 1065 compares the image characteristic extracted by the image-characteristic extracting section 1064 with the image characteristic set by the image-characteristic setting section 1062. In accordance with the result of comparison performed in the image-characteristic comparing section 1065, the illumination-characteristic correcting section 1066 generates an illumination-characteristic correcting signal for reducing the difference between the image characteristic extracted by the image-characteristic extracting section 1064 and the image characteristic set by the image-characteristic setting section 1062. The illumination-characteristic correcting signal is input to the light-source module controlling section 1042. Upon receiving this signal, the light-source module controlling section 1042 changes the intensity of the illumination light.

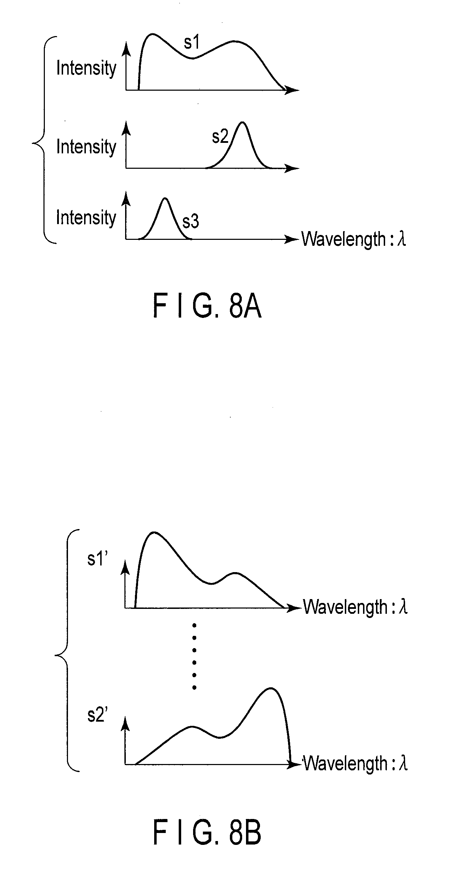

Assume that the light sources s1, s2 and s3 having such spectrum distributions as shown in FIG. 8A are used to emphasize a particular wavelength band as in the first embodiment. Then, in the third embodiment, the illumination-characteristic correcting signal is input from the illumination-characteristic correcting section 1066 to the light-source module controlling section 1042 in order to eliminate the difference between the image characteristic set by the image-characteristic setting section 1062 and the image characteristic extracted by the image-characteristic extracting section 1064. In accordance with the illumination-characteristic correcting signal, the light-source module controlling section 1042 changes the characteristic of the illumination light. If the output intensity of the light source s3 is higher than that of the light source s1, the color temperature can be raised (thereby emphasizing blue) as seen from the spectrum distribution s1' shown in FIG. 8B. If the output intensity of the light source s2 is increased, the color temperature can be lowered (thereby emphasizing red) as seen from the spectrum distribution s2' shown in FIG. 8B. As the illumination characteristics are repeatedly corrected in this way, image data having desirable characteristics can be acquired.

As described above, this embodiment has the illumination-characteristic correcting section 1066, which performs feedback control on the illuminating unit 104. Image data having desirable characteristics can be acquired can therefore be acquired.

In this embodiment, the color temperature is feedback-controlled as described above. In order to control, for example, the size of the illuminated area, a plurality of illumination optical systems different in light distribution pattern may be used to feedback-control the size of the illuminated area.

### [Fourth Embodiment]

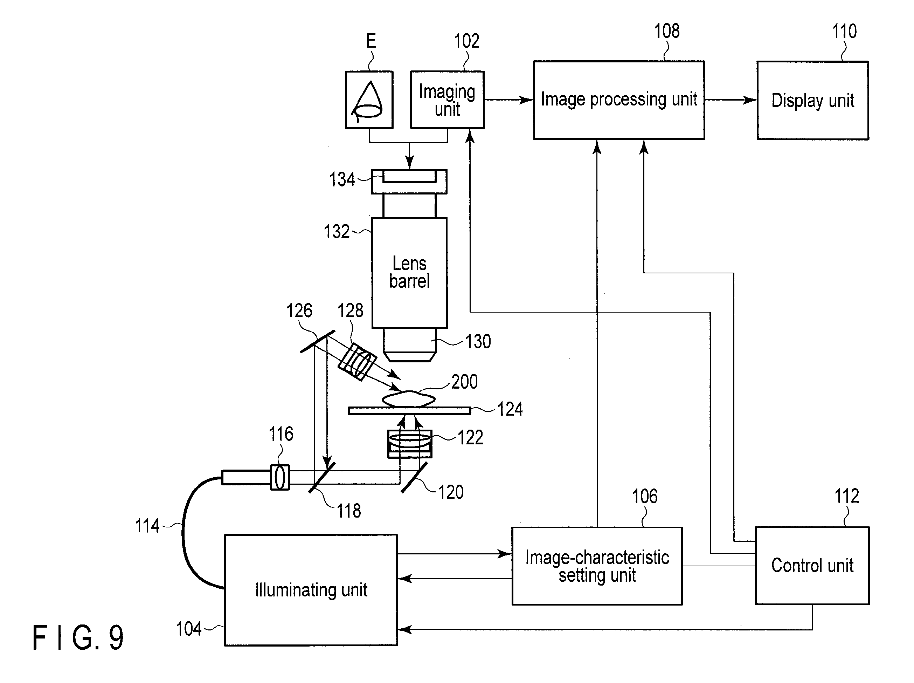

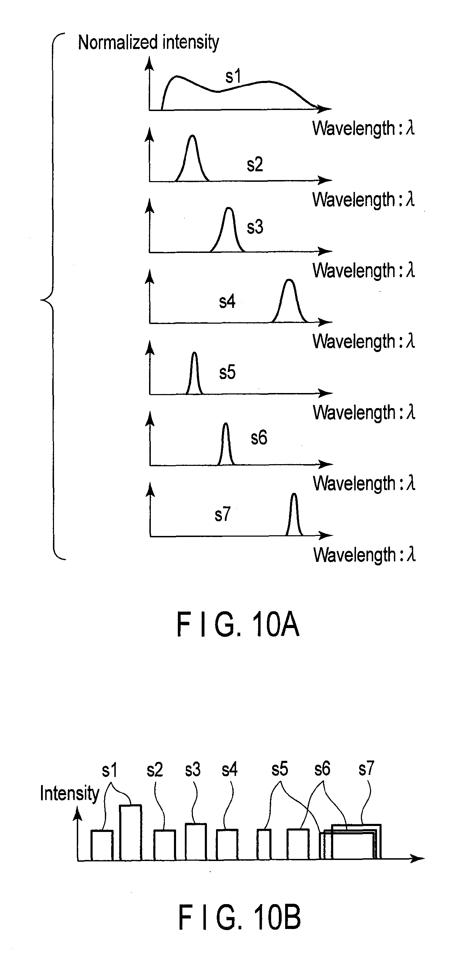

The fourth embodiment of the invention will be described. The fourth embodiment is a microscope apparatus that incorporates the imaging apparatus 100 according to any embodiment described above. FIG. 9 shows the microscope. FIG. 10A shows the spectrum distributions of the light sources used, and FIG. 10B shows the timing of light emission of the light sources. The microscope apparatus applies illumination light, at high density, to a very small object. The microscope is therefore one of representative examples that can provide an "environment in which the external light applied to the object 200 is negligibly weak, in effect, with respect to the illumination light applied to the object 200 from an illuminating means."

In the imaging apparatus of FIG. 9, the movable mirror 118 can be pulled from the optical path of illumination light. While the movable mirror 118 remains outside the optical path of illumination light, the illumination light emitted from the illuminating unit 104 is applied through a light guide 114 to a collimate optical system 116. The collimate optical system 116 converts the illumination light to parallel light. The parallel light travels through a rising mirror 120 and an illumination optical system 122 and is applied, as transmitting illumination light, to an object 200 to be observed through the microscope apparatus.

While the movable mirror 118 remains inserted in the optical path of illumination light, the illumination light emitted from the illuminating unit 104 is reflected by the movable mirror 118. The illumination light is then reflected by a turn-back mirror 126, travels through an illumination optical system 128 and is applied to the object 200.

The illumination light applied to the object 200 is reflected from, passes through, is scattered in, and is diffracted in, the object 200, and enters an objective optical system 130, together with fluorescent light. The illumination light reflected by the object 200 travels through the objective optical system 130 and a lens barrel 132, emerges from an ocular optical system, or imaging optical system 134. The image of the object 200 is thereby perceived by the observer's eyes E or imaged by the imaging unit 102.

The fourth embodiment uses seven light sources having such different spectrum distributions as shown in FIG. 10A. These light sources emit light at such timing and in such intensity as shown in FIG. 10B. As a result, white images or microscope images in a specific wavelength range can be acquired by the method described above as in any embodiment described above. Further, the microscope images acquired can be synthesized to provide various desirable microscope images.

The imaging apparatus shown in FIG. 9 has the configuration of the second embodiment. Needless to say, the imaging apparatus may have the configuration of the first embodiment or the configuration of the third embodiment.

### [Fifth Embodiment]

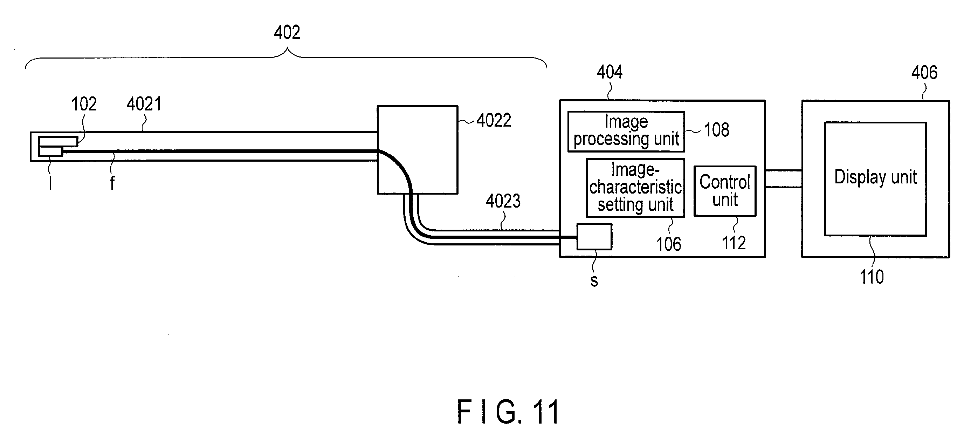

The fifth embodiment of the invention will be described. The fifth embodiment is an endoscope apparatus that incorporates the imaging apparatus 100 according to any embodiment described above. The basic configuration of the endoscope apparatus is shown in FIG. 11. The endoscope apparatus applies illumination light to an object in a living subject or a laying pipe, almost not influenced by external light. The endoscope is therefore considered another example that provides an "environment in which the external light applied to the object is negligibly weak, in effect, with respect to the illumination light applied to the object from an illuminating means," even if the external-light shielding member 300 is not used.

The endoscope apparatus shown in FIG. 11 comprises a main unit control device 404 having an image processing function and a light source function, a display device 406 having a display unit 110, and an insertion unit 402. The display device 406 and insertion unit 402 are connected to the main unit control device 404. The insertion unit 402 is composed of a distal flexible section 4021, a scope manipulation section 4022, and a proximal flexible section 4023. The main unit control device 404 incorporates an image processing unit 108, an image-characteristic setting unit 106, a control unit 112, and a light source unit s. The light source unit s is a part of an illuminating unit. The output end of the light source s is connected to a light guiding path f composed of, for example, an optical fiber. The light emitted from the light source s is guided toward the distal flexible section 4021 of the insertion unit 402, and is applied, as needed, to the object through an illumination optical system 1.

The light source s, light guiding path f and illumination optical system 1 may have, for example, the configurations shown in FIG. 2. In this case, the light beams emitted from light sources s1, s2 and s3 are combined by a combiner in one light guiding path. The combined light beam is applied to the object through a common illumination-light optical system. The light beam emitted from the light source s4 is guided by another light guiding path and is applied to the object through an illumination-light optical system.

The illumination light reflected from, scattered in, and diffracted in, the object, and fluorescent light enter the imaging unit 102 provided in the distal flexible section 4021. The imaging unit 102 generates image data from the illumination light. The image data is transmitted by a signal transmitting means (not shown) provided in the insertion unit 402, to the image processing unit 108, and is stored in the frame memory 1081 provided in the image processing unit 108 of the main unit control device 404. As in any embodiment described above, the image synthesizing unit 1082 and display-characteristic adjusting section 1083 perform processes, and the display unit 110 displays the image.

The insertion unit 402 of the endoscope is flexible, is shaped like a tube and incorporates some electronic circuits. A variable light source module that can be mounted on the insertion unit 402 will be described below.

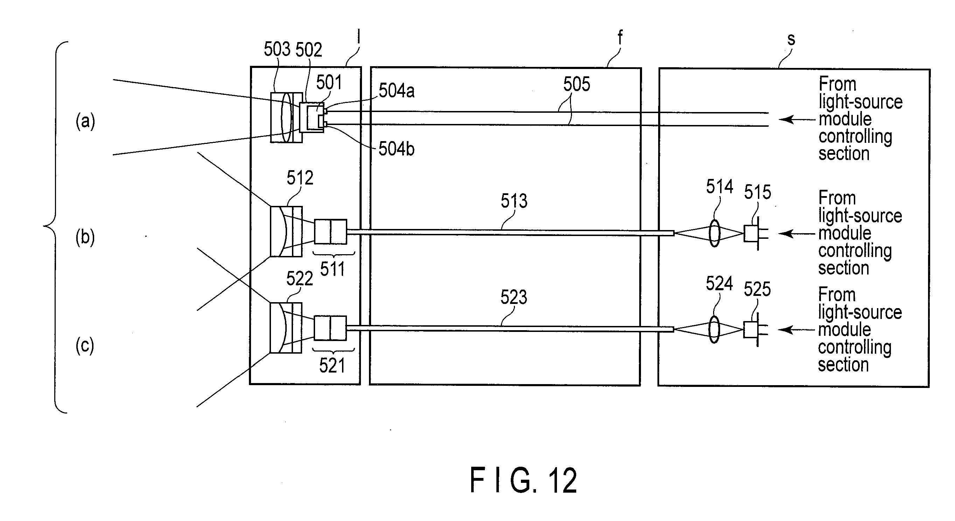

As shown in FIG. 12, the variable light source module has three light modules, (a), (b) and (c). The module (a) comprises a light source and a convex lens. The module (b) comprises a light source, an optical fiber, a phosphor member and a convex lens. The module (c) comprises a light source, an optical fiber, a light diffusing member and a convex lens.

In the light source module (a), a light source 501, a phosphor member 502 and a convex lens 503 are arranged in the distal flexible section 4021. The light source 501 is, for example, an LED chip or a laser chip. The light source 501 has driving electrodes 504a and 504b, which are connected to an electric wire 505. The electric wire 505 is connected to the light-source module controlling section 1042.

The light-source module controlling section 1042 generates a drive current. The drive current is supplied by the electric wire 505 to the light source 501 through the driving electrodes 504a and 504b. The phosphor member 502 converts the light emitted from the light source 501 to light of a desirable wavelength. The light so converted is applied to the object through the convex lens 503.

In the light source module (b) shown in FIG. 12, a phosphor unit 511 and a concave lens 512 are arranged in the distal flexible section 4021. The phosphor unit 511 has a laser-beam diversion control member and a phosphor member. The laser-beam diversion control member is a transparent columnar member. The output end of an optical fiber 513 is connected to the diffusion member of the phosphor unit 511. A coupling lens 514 and a light source 515 are arranged at the input end of the optical fiber 513. The light source 515 is connected to the light-source module controlling section 1042. The light source 515 is, for example, a laser chip.

Controlled by the light-source module controlling section 1042, the light source 515 emits excitation light, which is applied through a coupling lens 514 to the optical fiber 513. The optical fiber 513 guides the excitation light to the phosphor unit 511. In the phosphor unit 511, the laser-beam diversion control member makes the excitation light diverge. The excitation light diverged is applied to the phosphor member. The phosphor member changes the wavelength of the light to a desirable wavelength. The light so changed in wavelength is output from the phosphor unit 511 and applied to the object through the concave lens 512. Assume that the excitation light emitted from the laser chip has a wavelength equivalent to purple, and that the light emitted from the phosphor member has a wavelength equivalent to red or blue. Then, the light applied to the object is either red light having spectrum s2 shown in FIG. 10A or blue light having spectrum s4 shown in FIG. 10A.

In the light source module (c) shown in FIG. 12, a diffusion unit 521 and a concave lens 522 are arranged in the distal flexible section 4021. The diffusion unit 521 has a laser-beam diversion control member and a diffusion member. The laser-beam diversion control member is a transparent columnar member. The output end of an optical fiber 523 is connected to the diffusion member of the phosphor unit 521. A coupling lens 524 and a light source 525 are arranged at the input end of the optical fiber 523. The light source 525 is connected to the light-source module controlling section 1042. The light source 525 is, for example, a laser chip.

Controlled by the light-source module controlling section 1042, the light source 525 emits excitation light, which is applied through a coupling lens 524 to the optical fiber 523. The optical fiber 523 guides the excitation light to the diffusion unit 521. In the diffusion unit 521, the laser-beam diversion control member makes the excitation light diverge. The excitation light diverged is applied to a diffusion member and then applied to the object through the concave lens 522. Since the laser beam has a very narrow spectrum, the light applied to the object has, for example, spectrum s5, s6 or s7 shown in FIG. 10A.

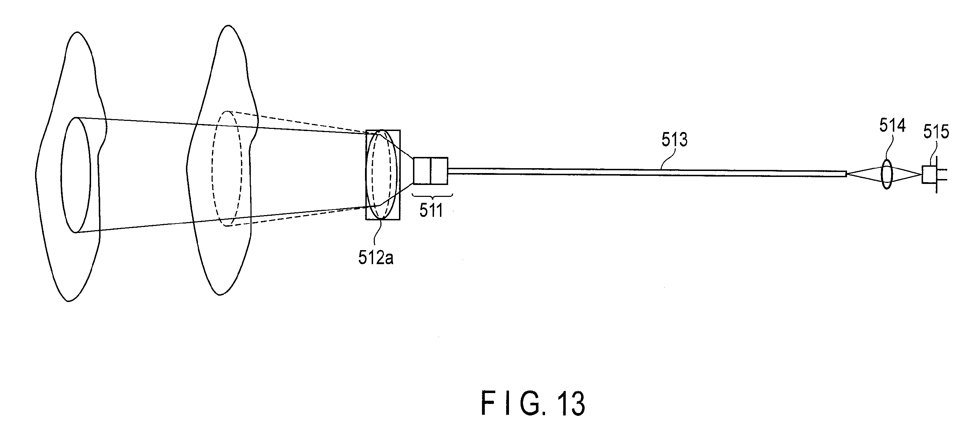

The light source module shown in FIG. 13 has a combination of variable focus lenses, and can change the size of the illuminated area. More specifically, the module has a configuration different from that shown in (b) in FIG. 12, in that a variable focus lens 512a is used in place of the concave lens 512.

In the light source module of FIG. 13, the focal distance of the variable focus lens 512a is changed to change the size of the area illuminated by one light source.

In the configuration of the fifth embodiment, too, the image-characteristic setting unit 106 sets the illuminating unit 104 and image processing unit 108, enabling the image-characteristic setting unit 106 to make the imaging unit 102 acquire image data having desirable image characteristics. Hence, complex image processing need not be performed on the image data acquired in the imaging unit 102.

## Claims

1. An imaging apparatus comprising:
an imaging unit configured to image an object to acquire image data about the object;
an illuminating unit including a light source configured to apply, to the object, a plurality of illumination light beams different in optical characteristics; and
an image-characteristic setting unit configured to set image characteristics of the image data the imaging unit should acquire, to refer to light source characteristic information including at least one of light-intensity control characteristic data, light-distribution pattern characteristic data, spectrum distribution characteristic data and light-polarization characteristic data, and to set to the illuminating unit, intensity, distribution pattern, spectrum distribution or polarization characteristic of at least one illumination light beam, thereby enabling the imaging unit to acquire effectively image data having the image characteristics set.

2. The imaging apparatus according to claim 1, wherein the image-characteristic setting unit sets the illuminating unit, causing the illuminating unit to switch at least one of the intensity, distribution pattern, spectrum distribution and polarization characteristic within a time shorter than a one-frame imaging time of the imaging unit.

3. The imaging apparatus according to claim 1 or 2, wherein the image-characteristic setting unit sets, to the illuminating unit, at least one of the intensity, distribution pattern, spectrum distribution and polarization characteristic in the one-frame imaging time of the imaging unit, and
the apparatus further comprises an image processing unit configured to synthesize a plurality of images acquired for each frame in the imaging unit.

4. The imaging apparatus according to claim 1, wherein the image-characteristic setting unit further comprises:
an image-characteristic extracting section configured to extract the image characteristic from the image acquired in the imaging unit;
an image-characteristic comparing section configured to compare the image characteristic extracted by the image-characteristic extracting section with the image characteristic set by the image-characteristic setting unit; and
an illumination-characteristic correcting section configured to correct at least one of the intensity, distribution pattern, spectrum distribution and polarization characteristic of the illumination light emitted by the illuminating unit.

5. The imaging apparatus according to claim 1, wherein the image characteristics change with time, and the image-characteristic setting unit refers to the light source characteristic information and changes with time, at least one of the intensity, distribution pattern, spectrum distribution and polarization characteristic of the illumination light, thereby enabling the imaging unit to acquire effectively the image having the image characteristics set.

6. The imaging apparatus according to claim 1,
wherein the image characteristics include a dynamic range of brightness for the image acquired in the imaging unit;
the image-characteristic setting unit sets the illuminating unit, causing the illuminating unit to emit the illumination light in a first intensity at first time and in a second intensity at second time if the dynamic range is set;
the imaging unit acquires a first image at the first time and a second image at the second time; and
the apparatus further comprises an image processing unit configured to synthesize the first image and the second image to acquire a synthesized image having the dynamic range that has been set.

7. The imaging apparatus according to claim 1,
wherein the image characteristics include a wavelength band to emphasize or suppress in the image acquired by the imaging unit; and
the image-characteristic setting unit first refers to the light source characteristic information and then sets the illuminating unit, causing the illuminating unit to synthesize illumination light beams different in spectrum distribution, thereby generating illumination light of the wavelength band to emphasize or suppress in the image acquired by the imaging unit.

8. The imaging apparatus according to claim 1,
wherein the image characteristics include a wavelength band to emphasize or suppress in the image acquired by the imaging unit;
the image-characteristic setting unit first refers to the light source characteristic information and then sets the illuminating unit, causing the illuminating unit to switch the spectrum of the illumination light with time;
the imaging unit acquires a plurality of images when the spectrum of the illumination light is switched; and
the apparatus further comprises an image processing unit configured to synthesize the images acquired to generate an image in which the wavelength band is emphasized or suppressed.

9. The imaging apparatus according to claim 1,
wherein the image characteristics include an object area to emphasize in brightness in the image acquired by the imaging unit; and
the image-characteristic setting unit first refers to the light source characteristic information and then sets the illuminating unit, causing the illuminating unit to apply the illumination light to the object area.

10. The imaging apparatus according to claim 1, further comprising a display unit configured to display at least one of information selected from a group consisting of information representing image characteristic, information representing light source characteristic and information representing the setting of the illuminating unit set by the image-characteristic setting unit, together with the image acquired by the imaging unit.

11. The imaging apparatus according to claim 1, wherein the light sources provided in the illuminating unit are a combination of a light source and an illumination optical system, a combination of a light source, a light guiding path, a phosphor member and an illumination optical system, a combination of a light source, a light guiding path and an illumination optical system, a combination of a light source and a variable-power optical system, or a combination of a light source and a polarization control optical system.

12. The imaging apparatus according to claim 1, wherein the imaging unit acquires an image and the illuminating unit illuminates the object in an environment where the external light applied to the object is, in effect, negligibly weak with respect to the illumination light applied to the object from the illuminating unit, and where the external light is prevented from entering the imaging unit, and any component of the external light can be canceled from the image acquired by the imaging unit or any component of the illumination light can be extracted.

13. A microscope apparatus comprising the imaging apparatus according to claim 1.

14. An endoscope apparatus comprising the imaging apparatus according to claim 1.
